# EUROPEAN PATENT SPECIFICATION

(11) **EP 1 310 267 B1**
(45) Date of publication and mention of the grant of the patent: **16.01.2008**
(21) Application number: 02425677.8
(22) Date of filing: 06.11.2002
(51) Int. Cl.: A61M 1/00

(54) **Infusion-suction cassette with two suction-pumps**
Spül- und Saugkassette mit zwei Saugpumpen
Cassette pour succion-irrigation avec deux pompes de succion

(30) Priority: 09.11.2001 IT RM20010669
(43) Date of publication of application: 14.05.2003
(73) Proprietor: Optikon 2000 S.p.a., 00138 Roma (IT)
(72) Inventor: Angelini, Giampiero, Optikon 2000 S.p.A., 00138 Roma (IT); Eibenschutz, Marco, Optikon 2000 S.p.A., 00138 Roma (IT)
(74) Representative: Iannone, Carlo Luigi

(56) References cited:
- WO-A-93/17729
- US-A- 4 758 238
- US-A- 6 083 195

## Description

The present invention relates to an infusion - suction (I/S) cassette provided with suction system having both a peristaltic or volumetric pump and a pressure sensitive (or Venturi) pump.

As it is well known, suction systems for ocular surgical instruments are provided with pump suitable to create vacuum up to a preestablished value set by the surgeon.

At present, various pumps are employed, said pump, generally speaking, being included in two groups, namely, volumetric kind pumps and pressure sensitive kind pumps.

In the volumetric kind pumps, such as peristaltic and gear pumps, the rotation of the pump induces the displacement of a liquid volume proportional to the rotation speed. Vacuum growths to the preestablished value only in case the suction opening is occluded, and therefore said value must be only intended as a safety value.

In the pressure sensitive kind pumps, such as Venturi effect pump, it is instead generated a vacuum at the wished level, independently from the occlusion of the suction opening.

Practically, while in the first kind of pumps the primary parameters is the volume of the liquid with respect to the time unit (flow) and the vacuum is a parameter depending on the impedance of the suction circuit, in the second group pumps the primary parameter is the vacuum level and the flow has a value depending on the impedance of the suction circuit.

The different behaviour of the two pump kinds is reflected in a different behaviour when used in the ophthalmic surgery, making the two systems preferred on the basis of the kind of surgical intervention or of the operation phase, or even according to the operator preferences.

For example, when removing cataracts having a low hardness, not being necessary reaching very high vacuum levels, it can be preferable to use a Venturi kind pump, due to the better performances in following masses of the nucleus and bringing them close to the tip of the phaco-emulsifier.

Furthermore, in the vitreous surgeon, case for which surgical instruments having very high impedance to the fluid passage, it is still preferred to use the Venturi pump since, due to the impendances, even with very high vacuum levels, they are not generated high suction flows and the Venturi pump does not have vacuum micro-fluctuations, that are typical of the action of the peristaltic pump rollers.

On the contrary, during the phaco-emulsification of the hard cataracts, requiring very high vacuum levels, Venturi pump has the disadvantage of creating, due to the low impedance of the phaco-emulsifier tip, too high flow levels that cannot be balanced by the irrigation flow while the peristaltic pump can be adjusted on a moderate flow and the vacuum brings to the wished value only when the surgical tip is occluded by the mass to be sucked (thus without generating liquid flow).

Until some years ago, machines for the ophthalmic surgery (cataract and retina) were divided into two groups:
- machines provided with Venturi pump, or in any case with volumetric pump;
- machines provided with Venturi pump, or in any case with pressure sensitive pump.

The first kind of machines is used more often, but not exclusively, for the cataract surgery, while the second kind is mainly used for the vitreous surgery in the eye rear segment.

More recently, some companies, among which Bausch & Lomb, have realised machines wherein, replacing the cassette containing disposable tubes, it is possible to operate with the peristaltic or Venturi system.

Surgeon must decide, before the intervention, the kind of pump he wishes to use and choose the suitable infusion - suction cassette (I/S).

This solution is a remarkable improvement with respect to the prior art.

However, it is unsolved the problem of needing two different kind of disposable cassettes.

Furthermore, by this solution, it is not possible, or in any case difficult, to change the kind of cassette and pump during the surgical intervention, for example in case the cataract to be removed is harder than expected before starting the intervention.

In WO-A-93-17729 is described a "System and apparatus for controlling fluid flow from a surgical handpiece" also including as well a peristaltic- and a venturi pump.

In view of the above, it is well evident the advantage of having available a I/S cassette as suggested by the present invention, having the feature of providing at the same time both the suction system by the peristaltic pump and by the Venturi pump.

By the solution according to the present invention, surgeon can switch from a suction system to another one, even more than once, also during the surgery intervention, by a simple manoeuvring and without replacing any part.

Further object of the present invention is that of realising the I/S cassette in such a way that it does not involve further costs with respect to a standard I/S cassette, i.e. an only Venturi or peristaltic cassette.

Still another object of the present invention is that of providing a disposable I/S cassette, system that is today often preferred due to the increase of the costs and of the complexity of the cleaning and resterilisation systems of the re-used materials due to the prevention of the diffusion through surgical way of new diseases such as AIDS and Creutzfeldt Jakob disease.

Further object of the present invention is that of realising an I/S cassette simple to be assembled on the machine, and that does not require complex manoeuvring or in any case requiring a proper interpretation by the operator, in order to avoid possible errors.

It is therefore specific object of the present invention an infusion-suction (I/S) cassette adapted to cooperate with a system having booth a peristaltic or volumetric pump and a pressure sensitive or Venturi pump, the cassette being defined in the claims.

Preferably, according to the invention, said pressure sensitive pump is a Venturi pump.

Still according to an embodiment of the invention, said first and second valves along the irrigation line can be of the membrane kind.

Furthermore, according to an embodiment of the invention, said valves can be pinch valves or of any other kind suitable to intercept the passage of sterile fluids.

Always according to the invention, said cassette is a disposable cassette.

Furthermore, according to an embodiment of the invention, inner channels of the cassette are integrally moulded in the same cassette, or they are comprised of lengths of PVC or other material tube, suitably blocked in a proper position within the cassette.

Further, in the cassette can be provided a membrane coupling between the suction line and the vacuum sensor.

The present invention will be now described, for illustrative but not limitative purposes, according to its preferred embodiments, with particular reference to the figures of the enclosed drawings, wherein:
figure 1 shows a peristaltic pump;
figure 2 shows a suction system with Venturi pump;
figure 3 schematically shows the combined system according to the invention;
figure 4 shows a lateral view of a possible use of the cassette according to the invention; and
figure 5 is a perspective view of the combined pump of figure 4.

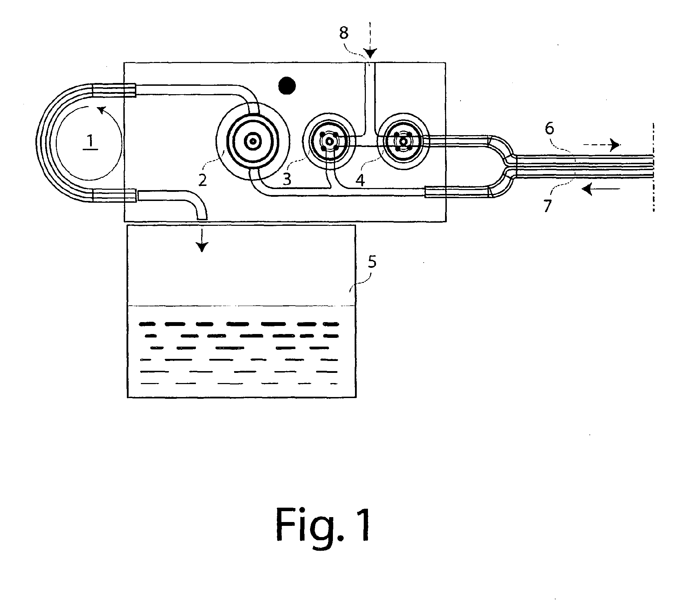

Observing first figure 1, it is shown a peristaltic pump 1 that, rotating in a anti-clockwise direction, sucks from the line (tube) 7 fluids coming from the surgical handpiece.

Vacuum sensor 2, provided in the control panel of the machine and coupled to the vacuum line of the I/S cassette, preferably in a sterile way, allows stopping the pump 1 when it is reached the vacuum value set by the surgeon.

Water sucked is disposed of in a collection tank 5, at the outlet of the pump that can be, or not, of the rigid kind (collection tank or bag).

The sterile balanced salt solution coming from the line 8 is directed to the surgical handpiece through the line 6 to balance the sucked fluids. Valve along line 8 allows to the surgeon to suspend the irrigation flow when it is necessary.

Valve 3 allows to introduce the fresh salt solution within the suction line, in order to instantaneously balance the pressure (vacuum) within the aspiration line when the surgeon wishes interrupting the sucking action (venting), or to reverse the flow direction within the line 7 in order to release erroneously sucked parts.

All the parts indicated in figure 1, excluding the rotor of the peristaltic or in any case volumetric pump, of the active element of the vacuum sensor, and of the solenoids (motors) of the valves intercepting fluids, are part of the I/S cassette and can be disposable.

Also the part in touch with fluids of the interception valves (pinch or membrane valves) usually is a part of the I/S cassette, that is disposed at the end of the intervention.

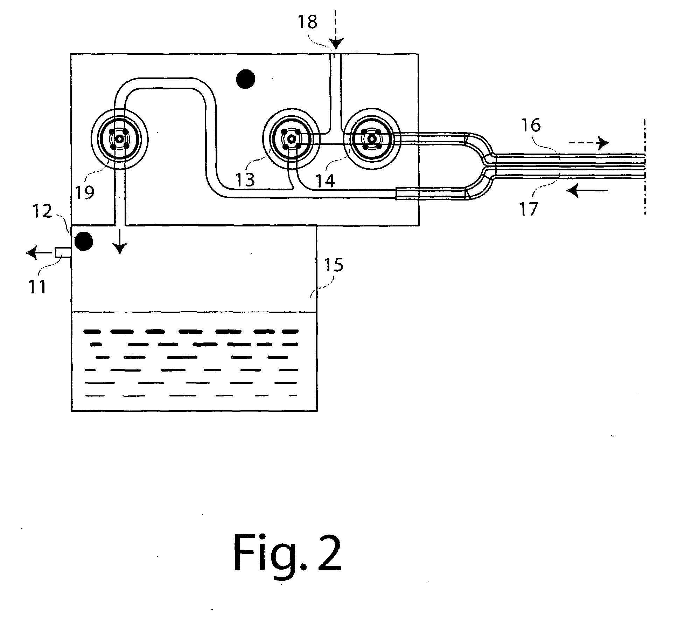

Making reference to figure 2, it is shown a Venturi pump, generically indicated by reference number 11, creating vacuum within the rigid tank connected to the line (tube) 17 and removes fluids through the surgical handpiece.

Vacuum sensor 12, provided in the control panel of the machine reads the vacuum level within the collection tank 15. Said solution usually is preferred in the Venturi system, since it prevents the needing of a sterile coupling with the line 17.

Since Venturi pump 11, when not activated, behaves as a atmosphere vent line, it is necessary to introduce the valve 19 in order to block the "passive" suction phenomenon: the sterile balanced salt solution, coming from the line 18, is directed to the surgical handpiece through the line 16 bringing the eye at a positive pressure, in case the line 17 is let opened at the other end (Venturi), a continuous flow is generated from the supply 18 to the tank 15.

The object and the operation of the valves 13 and 14 is absolutely identical to that described for the peristaltic system, making reference to figure 1.

As for the peristaltic cassette of figure 1, all the parts shown in figure 2, with the exception of the Venturi pump 11, of the vacuum sensor 12 and of the valve 13, 14 solenoids intercepting the fluids, are part of the I/S cassette and can be disposable.

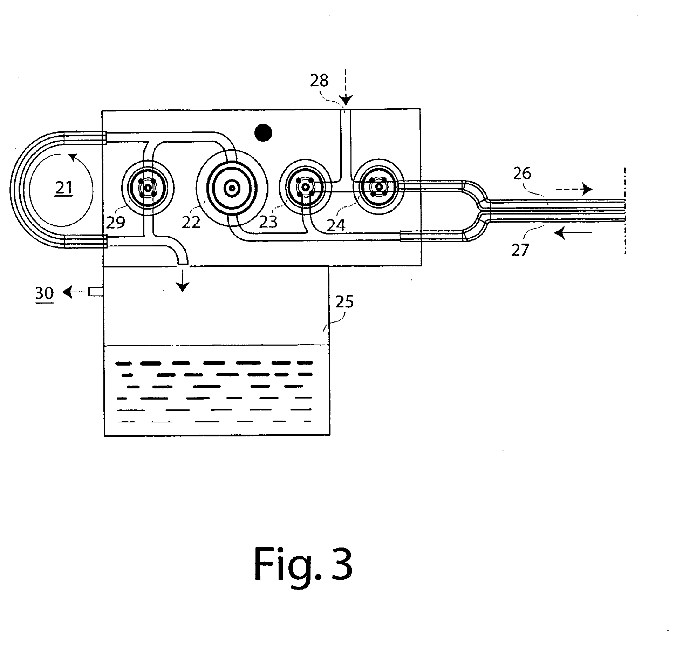

Coming now to observe figure 3 of the enclosed drawings, it is shown an I/S cassette 20 according to the invention, comprising both a peristaltic system 21 and a Venturi system 30.

If solution of figure 3 is compared with the solution of figure 1, it is noted that, in case valve 29 remains closed and Venturi pump 30 is deactivated (we remember that it behaves as an open line), the two systems behaves in an absolutely identical way, i.e. cassette 20 according to the invention works as a peristaltic pump. Each part has the same functions of the corresponding part of the other system.

If the peristaltic pump 21 is stopped, it keeps the tube squeezed (closed) about its impeller and the system is practically identical to what is described with reference to figure 2 (Venturi pump), with the sole difference (improvement) of carrying out the vacuum reading within the suction line 27 rather than within the collection tank 25.

Cassette 20 can thus operate either as a suction system by the peristaltic pump 21 and as Venturi system 30, being it possible instantaneously switching from a mode to the other one by the operation of the pump 21 and of the valve 29.

Under the costs point of view, pumps 21 and 30 are placed in the control panel and the sole adding to the Venturi kind cassette of figure 2 to obtain the dual-mode cassette 20 according to the invention, is the tube length 31 that should be introduced about the rotor of the peristaltic pump 21.

The only variation with respect to the cassette of figure 1 is the presence of the valve 29, that in any case is only partially realised (moulded) in the cassette (in fact, solenoid is a part of the control panel).

Costs of cassette 20 according to the invention are thus practically identical to the costs of an only peristaltic or Venturi cassette.

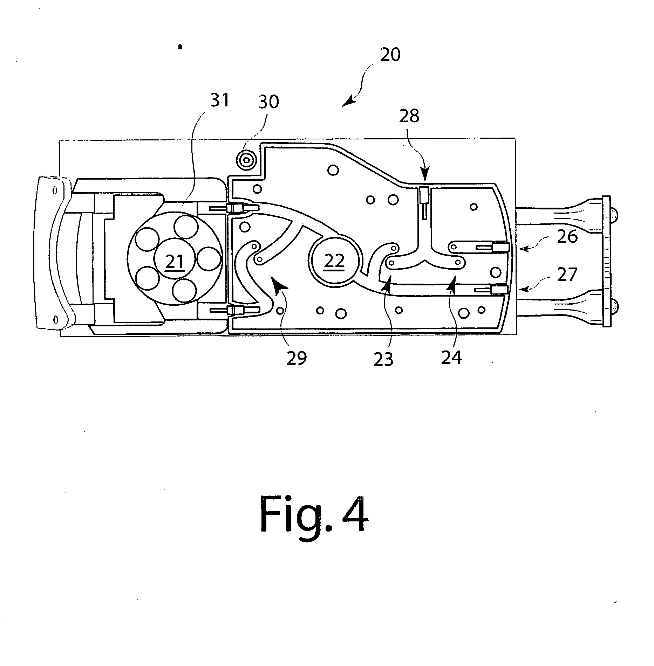

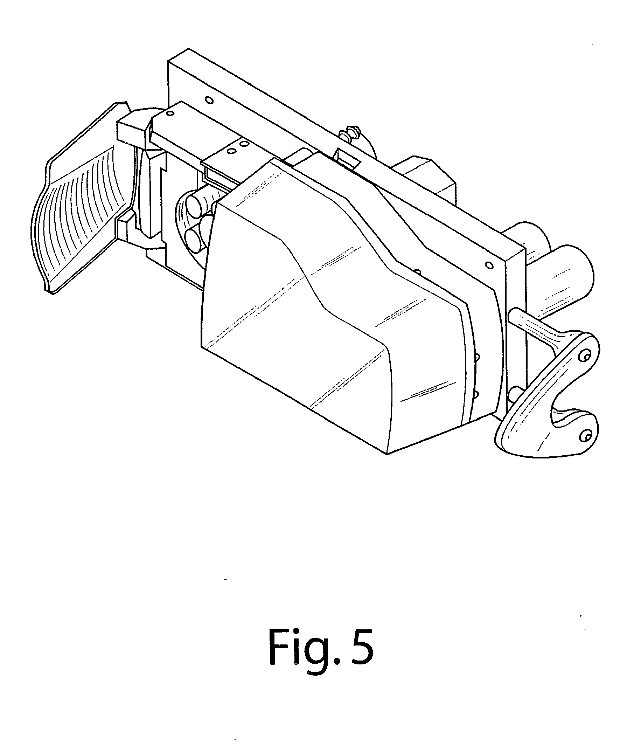

In figures 4 and 5, it is shown a possible realisation of the system providing the cassette 20 according to the invention. Particularly, in figure 4 the collection tank has been removed to show the channels and the inside part.

In the figure they are not shown the lines (PVC or other material tubes) connecting to the sterile balanced salt solution irrigation inlet 28, the outlet 26 to the irrigation line of the handpiece and the entrance 27 to the suction line of the same.

In the embodiment proposed, channel inside the cassette 20 is integrally moulded within the same. It would be possible to replace the channel realised by moulding with PVC or other material tube lengths, suitably blocked in a proper position within the cassette 20.

In the suggested solution, valves 23, 24 are of the membrane kind. It is in any case possible also to use pinch valves or any other kind suitable to intercept the passage of sterile fluids.

By the numeral reference 22 it is indicated a possible membrane coupling between the suction line 27 and the vacuum sensor 23 in the control panel, to prevent the contact among sucked fluids and the same sensor 23 (that is not sterile).

To obtain the same result, it is possible to use bacterio-static filters or analogous systems.

In figure 5 it is shown a possible realisation of the pump coupled to its support plate (on the control panel). It is possible to see actuation solenoids of the valves placed within the control panel. It is also shown the collection tank 25.

The present invention has been described for illustrative but not limitative purposes, according to its preferred embodiments, but it is to be understood that modifications and/or changes can be introduced by those skilled in the art without departing from the relevant scope as defined in the enclosed claims.

## Claims

1. An infusion-suction (I/S) cassette (20) adapted to cooperate with a system having booth a peristaltic or volumetric pump (21) and a pressure sensitive or Venturi pump (30), the cassette comprising a fluid suction line (27) attachable to a surgical handpiece, a feeding line (28) for a sterile irrigation solution, a first valve (23) between the feeding line (28) and the fluid suction line (27) and a second valve (24) between the feeding line (28) and a handpiece irrigation line (26) for interruption of the passage of the irrigation solution, wherein said cassette further provides a vacuum sensor (22), a valve (29) capable of blocking passive suction through the Venturi pump when the pump is deactivated, a collection tank (25), the handpiece irrigation line (26), a line around the peristaltic or volumetric pump (21), to which line the suction line (27) is connected via the vacuum sensor (22), the pressure sensitive pump being connectable to the suction fine via the valve for blocking passive suction and via the vacuum sensor (22), where activation of the peristaltic or volumetric pump (21) involves closing of said passive-suction blocking valve (29) and also involves deactivation of said pressure sensitive pump (30), the activation of the pressure sensitive pump (30) involving opening of said passive-suction blocking valve and deactivation of the peristaltic or volumetric pump (21), causing the interruption of the line (31) around the peristaltic pump (21), and consequent deviation of the suction line through said passive suction blocking valve (29).

2. Infusion - suction (I/S) cassette according to claim 1, **characterised in that** said first (23) and second (24) valves along the irrigation line (28) are of the membrane kind.

3. Infusion-suction (I/S) cassette according to claim 1, **characterised in that** said valves (23,24) are pinch valves or of any other kind suitable to intercept the passage of sterile fluids.

4. Infusion - suction (I/S) cassette according to one of the preceding claims, **characterised in that** said cassette is a disposable cassette.

5. Infusion-suction (I/S) cassette according to one of the preceding claims, **characterised in that** the inner channels of the cassette are integrally moulded in the same cassette, or they are comprised of lengths of PVC or other material tube, suitably blocked in a proper position within the cassette.

6. Infusion-suction (I/S) cassette according to one of the preceding claims, **characterised in that** there is provided a membrane coupling between the suction line (27) and the vacuum sensor (22).

## Patentansprüche

1. Eine Infusions-Saug-(I/S)-Kassette (20), angepasst an die Zusammenarbeit mit einem System, das sowohl eine peristaltische oder volumetrische Pumpe (21) als auch eine druckempfindliche oder Venturi-Pumpe (30) besitzt, wobei die Kassette eine Fluidsaugleitung (27), die an einem medizinischen Handstück angebracht werden kann, eine Zuführungsleitung (28) für eine sterile Spüllösung, ein erstes Ventil (23) zwischen der Zuführungsleitung (28) und der Fluidsaugleitung (27) und ein zweites Ventil (24) zwischen der Zuführungsleitung (28) und einer Handstückspülleitung (26) zur Unterbrechung des Durchlaufs der Spüllösung umfasst, wobei die Kassette ferner bereitstellt: einen Vakuumsensor (22), ein Ventil (29), welches das passive Ansaugen durch die Venturi-Pumpe bei deaktivierter Pumpe blockieren kann, einen Sammeltank (25), die Handstückspülleitung (26), eine Leitung um die peristaltische oder volumetrische Pumpe (21), mit welcher Leitung die Saugleitung (27) über den Vakuumsensor (22) verbunden ist, wobei die druckempfindliche Pumpe über das Ventil zum Blockieren von passivem Ansaugen und über den Vakuumsensor (22) mit der Saugleitung verbunden werden kann, wobei die Aktivierung der peristaltischen oder volumetrischen Pumpe (21) sowohl das Schließen des Ventils zum Blockieren von passivem Ansaugen (29) als auch das Deaktivieren der druckempfindlichen Pumpe (30) einschließt, wobei die Aktivierung der druckempfindlichen Pumpe (30) das Öffnen des Ventils zum Blockieren von passivem Ansaugen und das Deaktivieren der peristaltischen oder volumetrischen Pumpe (21) einschließt, was zur Unterbrechung der Leitung (31) um die peristaltische Pumpe (21) und der folglichen Ablenkung der Saugleitung durch das Ventil zum Blockieren von passivem Ansaugen (29) führt.

2. Infusions-Saug-(I/S)-Kassette nach Anspruch 1, **dadurch gekennzeichnet, dass** das erste Ventil (23) und das zweite Ventil (24) entlang der Spülleitung (28) als Membrantyp ausgeführt sind.

3. Infusions-Saug-(I/S)-Kassette nach Anspruch 1, **dadurch gekennzeichnet, dass** die Ventile (23,24) Quetschventile sind oder als irgendein anderer Typ, der geeignet ist, den Durchlauf steriler Fluide zu unterbrechen, ausgeführt sind.

4. Infusions-Saug-(I/S)-Kassette nach einem der vorhergehenden Ansprüche, **dadurch gekennzeichnet, dass** die Kassette als Einwegkassette ausgeführt ist.

5. Infusions-Saug-(I/S)-Kassette nach einem der vorhergehenden Ansprüche, **dadurch gekennzeichnet, dass** die inneren Kanäle der Kassette integral in derselben Kassette geformt sind oder dass sie aus Rohrlängen aus PVC oder einem anderen Material bestehen, die angemessen in einer passenden Position in der Kassette blockiert sind.

6. Infusions-Saug-(I/S)-Kassette nach einem der vorhergehenden Ansprüche, **dadurch gekennzeichnet, dass** eine Membrankupplung zwischen der Saugleitung (27) und dem Vakuumsensor (22) bereitgestellt ist.

## Revendications

1. Cassette de perfusion-aspiration (P/A) (20) conçue pour coopérer avec un système comprenant une pompe péristaltique ou volumétrique (21) ainsi qu'une pompe sensible à la pression ou Venturi (30), la cassette comprenant une ligne d'aspiration de fluide (27) pouvant être raccordée à un instrument chirurgical, une ligne d'alimentation (28) pour une solution d'irrigation stérile, un premier robinet (23) entre la ligne d'alimentation (28) et la ligne d'aspiration de fluide (27) et un deuxième robinet (24) entre la ligne d'alimentation (28) et une ligne d'irrigation d'instrument (26), pour l'interruption du passage de la solution d'irrigation, ladite cassette comprenant, en outre, un détecteur de vide (22), un robinet (29) capable de bloquer une aspiration passive à travers la pompe Venturi lorsque la pompe est désactivée, un réservoir de récupération (25), la ligne d'irrigation d'instrument (26), une ligne s'étendant autour de la pompe péristaltique ou volumétrique (21), ligne à laquelle la ligne d'aspiration (27) est raccordée par l'intermédiaire du détecteur de vide (22), la pompe sensible à la pression pouvant être raccordée à la ligne d'aspiration par l'intermédiaire du robinet destiné à bloquer une aspiration passive et du détecteur de vide (22), où l'activation de la pompe péristaltique ou volumétrique (21) met en jeu la fermeture dudit robinet de blocage d'aspiration passive (29) ainsi que la désactivation de ladite pompe sensible à la pression (30), l'activation de la pompe sensible à la pression (30) mettant en jeu l'ouverture de dudit robinet de blocage d'aspiration passive et la désactivation de la pompe péristaltique ou volumétrique (21), qui provoque l'interruption de la ligne (31) s'étendant autour de la pompe péristaltique (21) et une déviation résultante de la ligne d'aspiration à travers ledit robinet de blocage d'aspiration passive (29).

2. Cassette de perfusion-aspiration (P/A) selon la revendication 1, **caractérisée en ce que** lesdits premier (23) et deuxième (24) robinets situés le long de la ligne d'irrigation (28) sont du type à membrane.

3. Cassette de perfusion-aspiration (P/A) selon la revendication 1, **caractérisée en ce que** lesdits robinets (23, 24) sont des robinets à manchon déformable ou d'un quelconque autre type approprié pour intercepter le passage de fluides stériles.

4. Cassette de perfusion-aspiration (P/A) selon l'une quelconque des revendications précédentes, **caractérisée en ce que** ladite cassette est une cassette jetable.

5. Cassette de perfusion-aspiration (P/A) selon l'une quelconque des revendications précédentes, **caractérisée en ce que** les canaux internes de la cassette sont moulés d'un seul tenant dans la même cassette, ou bien ils sont constitués de longueurs de tubes en PVC ou en un autre matériau, adéquatement maintenus dans une position correcte à l'intérieur de la cassette.

6. Cassette de perfusion-aspiration (P/A) selon l'une quelconque des revendications précédentes, **caractérisée en ce qu'**il est prévu un couplage à membrane entre la ligne d'aspiration (27) et le détecteur de vide (22).
